# FASCICULE DE BREVET EUROPEEN

(11) **EP 4 058 129 B1**
(45) Date de publication et mention de la délivrance du brevet: **25.11.2026**
(21) Numéro de dépôt: 20823892.3
(22) Date de dépôt: 13.11.2020
(51) Int. Cl.: A61M 60/863, A61M 60/17, A61M 60/237, A61M 60/865

(54) **ENSEMBLE DE POSE D'UNE POMPE CARDIAQUE ÉQUIPÉ D'UN DISPOSITIF D'ANCRAGE DE LA POMPE CARDIAQUE**
ANORDNUNG ZUM ANBRINGEN EINER HERZPUMPE WELCHE MIT EINER VORRICHTUNG ZUR VERANKERUNG DER HERZPUMPE VERSEHEN IST
ASSEMBLY FOR FITTING A CARDIAC PUMP PROVIDED WITH A DEVICE FOR ANCHORING THE CARDIAC PUMP

(30) Priorité: 15.11.2019 FR 1912799
(43) Date de publication de la demande: 21.09.2022
(73) Titulaire: Fineheart, 33600 Pessac (FR)
(72) Inventeur: COLLAS, Jérémy, 33200 BORDEAUX (FR); GARRIGUE, Stéphane, 33130 BEGLES (FR); BOUGERE, Emmanuel, 33600 PESSAC (FR)
(74) Mandataire: Plasseraud IP
(86) Numéro de dépôt international: PCT/FR2020/052083
(87) Numéro de publication internationale: WO 2021/094698

(56) Documents cités:
- EP-A1- 2 524 709
- WO-A1-2016/018434
- WO-A1-2018/052482
- FR-A1- 3 059 907
- US-A1- 2014 378 772

## Description

### Domaine technique

La présente divulgation concerne un dispositif d'ancrage d'une pompe cardiaque dans une ouverture d'une paroi ventriculaire d'un cœur battant ou d'un cœur non battant bénéficiant d'une circulation extra-corporelle. Un exemple de dispositif de l'art antérieur est décrit dans FR3059907A1.

Elle concerne également un ensemble de pose d'une pompe cardiaque intégrant un tel dispositif d'ancrage.

### Technique antérieure

L'insuffisance cardiaque (IC) est un état pathologique dans lequel le cœur d'un patient présente une incapacité à fournir un débit sanguin nécessaire aux besoins métaboliques de l'organisme.

Il est connu pour traiter l'insuffisance cardiaque, d'implanter un dispositif d'assistance ventriculaire (DAV), qui est une pompe cardiaque artificielle.

Cette pompe mécanique ne remplace pas le cœur qui continue à fonctionner, mais apporte une aide au ventricule affaibli afin d'accroître le débit sanguin de façon adaptée aux besoins de l'individu.

Cette assistance peut être temporaire dans l'attente d'un greffon disponible pour réaliser une transplantation cardiaque.

Cependant, on observe une proportion significative de patients qui ne recevront pas un tel greffon, soit parce qu'ils ne peuvent être candidats à une telle transplantation, par exemple en raison d'une insuffisance cardiaque sévère, soit parce qu'aucun greffon adapté n'est disponible pour ces patients.

Dans ce cas, l'assistance ventriculaire est utilisée en destination, c'est-à-dire que la pompe cardiaque artificielle est implantée à long terme.

Ces pompes cardiaques font donc l'objet d'intenses recherches visant à améliorer la qualité de vie des patients présentant une insuffisance cardiaque, ces pompes pouvant rester actives de plusieurs mois à plusieurs années.

De nombreuses avancées ont été réalisées et on connaît aujourd'hui des dispositifs d'assistance ventriculaire plus compacts, silencieux et présentant une durée de service accrue.

Les pompes cardiaques implantables de l'état de l'art sont ainsi typiquement équipées d'un moteur électrique intégré pour assurer leur fonctionnement, la vitesse de rotation de la pompe fournissant la force nécessaire pour faire circuler le sang depuis le ventricule affaibli vers la circulation corporelle.

On connaît également des systèmes d'implantation de telles pompes dans une ouverture d'une paroi ventriculaire.

Ces systèmes d'implantation comprennent généralement une portion tubulaire aux extrémités de laquelle sont placées, ou formées, des collerettes destinées à être plaquées chacune contre une face opposée de la paroi ventriculaire après introduction de la portion tubulaire dans une ouverture réalisée dans cette paroi ventriculaire avec un dispositif de carottage.

A titre d'exemple, on connaît une portion tubulaire creuse à l'extrémité proximale de laquelle est placée une première bride et à l'extrémité distale de laquelle est placée une membrane auto-expansible entre une première configuration, dite déformée, dans laquelle cette membrane présente une forme tubulaire, et une seconde configuration, dite initiale, dans laquelle elle définit une seconde bride. Cette configuration initiale est atteinte, lorsque cette extrémité distale est introduite à l'intérieur du cœur d'un patient.

De telles collerettes permettent ainsi de maintenir en position la portion tubulaire creuse qui définit alors un orifice traversant la paroi ventriculaire.

A l'extrémité, placée à l'extérieur du cœur, de cette portion tubulaire, est introduite une pompe cardiaque qui une fois installée, assure le renvoi du sang présent dans le ventricule vers la circulation corporelle.

Bien que donnant de bons résultats, ces systèmes d'implantation peuvent encore être améliorés.

Notamment, ils sont délicats à mettre en œuvre et à poser sur un cœur battant. Ils demandent une grande dextérité des praticiens réalisant l'intervention pour assurer la réussite de celle-ci, ce qui est source de stress.

Il existe donc un besoin pour un dispositif d'ancrage, dont la conception originale permette de surmonter les inconvénients de l'art antérieur exposés ci-dessus.

### Objet de l'invention

La présente invention est définie par les revendications annexées. Elle vise à pallier les inconvénients de l'art antérieur et à répondre aux contraintes ci-dessus énoncées en proposant un dispositif d'ancrage d'une pompe cardiaque, simple dans sa conception et dans son mode opératoire, réduisant la complexité de l'intervention chirurgicale tout en étant d'une grande fiabilité.

Un autre objet de la présente divulgation est un tel dispositif d'ancrage assurant une étanchéité totale entre l'intérieur et l'extérieur du cœur.

La présente invention vise un ensemble de pose d'une pompe cardiaque comportant un tel dispositif d'ancrage, cet ensemble permettant de réduire les pertes de sang pour le patient lors de l'intervention chirurgicale et d'accroître sa récupération.

### Exposé de l'invention

A cet effet, il est décrit un dispositif d'ancrage d'une pompe cardiaque dans une ouverture d'une paroi ventriculaire d'un cœur, comprenant un connecteur destiné à être monté sur cette paroi ventriculaire.
Selon l'invention,
- ce connecteur comprend une bride d'assemblage et une jupe de support, ou projection tubulaire, s'étendant à partir de cette bride d'assemblage, cette jupe de support étant destinée à être placée à l'extérieur du cœur lorsque la bride d'assemblage est assemblée à la paroi ventriculaire, ce connecteur définissant une première ouverture pour le passage d'une partie du corps d'un insert,
- cet insert étant entièrement rigide et définissant une seconde ouverture pour le passage du corps de la pompe cardiaque, cet insert comprenant une extrémité distale destinée à être placée à l'intérieur du cœur et une extrémité proximale destinée à être placée à l'extérieur du cœur, lorsque cet insert est monté sur le connecteur solidaire de la paroi ventriculaire, et
- au moins un élément de verrouillage/déverrouillage en position de l'insert par rapport au connecteur, lorsque cet insert a été introduit dans le connecteur.

Un tel dispositif d'ancrage d'une pompe cardiaque dans l'ouverture d'une paroi ventriculaire d'un cœur est encore connu sous le nom de bague d'ancrage, ou de dispositif de fixation, d'une pompe cardiaque.
Il est avantageusement entièrement réalisé dans un matériau biocompatible, non toxique et stérile.

Un tel dispositif est particulièrement adapté pour une intervention sur un cœur battant ou un cœur non battant bénéficiant d'une circulation extra-corporelle.

Ce dispositif d'ancrage peut ainsi être constitué simplement d'un connecteur pouvant être monté sur un cœur et définissant un orifice, d'un insert destiné à être porté par ce connecteur, en ayant une partie de son corps traversant une ouverture réalisée dans la paroi cardiaque, cette ouverture étant entourée par ce connecteur, un ou plusieurs éléments assurant le blocage en position de l'insert par rapport au connecteur lorsque cet insert a été monté sur ce connecteur solidarisé à cette paroi ventriculaire.

De manière avantageuse, un tel dispositif d'ancrage assure un confort accru pour l'équipe chirurgicale lors de l'intervention car ce dispositif d'ancrage est d'une pose particulièrement aisée. Il assure également une sécurité renforcée pour le patient, car une étanchéité totale est réalisée entre l'intérieur et l'extérieur du cœur du patient, du type valve unidirectionnelle bi ou tri-valvée, diaphragme ou encore clapet.

Dans le contexte de la présente invention, le terme « proximal » signifie la position la plus proche du professionnel de santé, ou du praticien, tandis que le terme « distal » doit ici être entendu comme signifiant le plus éloigné de ce professionnel. En d'autres termes, l'extrémité distale d'un élément est l'extrémité qui serait la première engagée dans le cœur d'un patient tandis que son extrémité proximale serait la dernière extrémité à y être engagée.

Selon un mode de réalisation particulier de ce dispositif d'ancrage, ce connecteur comprenant une bride de renfort placée à son extrémité proximale, l'insert comporte également une bride de renfort, ces brides de renfort étant destinées à être plaquées l'une contre l'autre lorsque l'insert est monté sur le connecteur.

Selon un autre mode de réalisation de ce dispositif d'ancrage, l'élément de verrouillage/déverrouillage est un élément de serrage traversant latéralement la bride de renfort du connecteur pour serrer le corps de l'insert ou un élément de fixation destiné à assembler ensemble les brides de renfort du connecteur et de l'insert.
Par exemple, cet élément de serrage peut être une vis.
Cette vis présentant un filetage externe, l'orifice traversant de la bride de renfort comporte un filetage complémentaire du filetage de la vis pour assurer l'engagement et le déplacement de cette vis, ainsi que l'étanchéité requise.
Alternativement, cet élément de serrage pourrait être un système de serrage à baïonnette dont les rainures seraient situées sur la surface interne de la jupe de support du connecteur.

Selon encore un autre mode de réalisation de ce dispositif d'ancrage, l'insert rigide présente une portion tubulaire dont la dimension longitudinale permet à l'extrémité distale de cet insert d'être placée à l'intérieur de la cavité cardiaque lorsque l'insert est monté sur le connecteur.

Selon encore un autre mode de réalisation de ce dispositif d'ancrage, le connecteur et/ou l'insert comportent une ou plusieurs rainures recevant chacune un joint d'étanchéité de préférence un joint torique.

Selon encore un autre mode de réalisation de ce dispositif d'ancrage, cet insert comprend un ou plusieurs éléments d'étanchéité pour assurer un assemblage étanche entre cet insert et le corps de pompe destiné à être inséré dans la seconde ouverture.

Selon encore un autre mode de réalisation de ce dispositif d'ancrage, la bride d'assemblage est réalisée dans un matériau permettant de promouvoir la croissance ou la fixation de cellules afin de renforcer son assemblage avec la paroi ventriculaire.
Ce matériau est également suturable ou agrafable.
A titre purement illustratif, cette bride d'assemblage est réalisée en Dacron^{®} ou Polytetrafluoroéthylène (PTFE).

Selon encore un autre mode de réalisation de ce dispositif d'ancrage, l'extrémité distale de l'insert est chanfreinée pour faciliter son insertion au travers d'un orifice réalisé dans la paroi ventriculaire

Selon encore un autre mode de réalisation de ce dispositif d'ancrage, l'insert comporte un élément de fixation pour bloquer en position le corps de pompe cardiaque lorsque cette dernière a été introduite dans la seconde ouverture.

La présente invention telle que revendiquée porte sur un ensemble de pose d'une pompe cardiaque sur un cœur comprenant un dispositif d'ancrage tel que décrit précédemment, et un fourreau amovible comportant un clapet anti-retour, ou vanne unidirectionnelle, ce fourreau étant destiné à être monté sur au moins l'extrémité proximale de l'insert pour prévenir toute perte de sang lors de l'insertion ou du retrait d'un outil, tel qu'un outil de carottage, ou de la pompe cardiaque.

Lors du retrait d'un outil de carottage, par exemple, cette valve unidirectionnelle se referme et redevient étanche. A titre d'exemple, il s'agit d'une membrane auto réparatrice, dite encore membrane auto-seal.

De manière avantageuse, ce fourreau amovible est réalisé dans un matériau flexible afin qu'il puisse recouvrir de manière étanche au moins l'extrémité proximale de l'insert.
Ce fourreau peut être inséré autour des extrémités proximales du connecteur et de l'insert pour recouvrir ces éléments.
De manière avantageuse, ce fourreau est réalisé en silicone ou tout autre matériau souple biocompatible tel qu'en Polyuréthane (PU) ou Polyétheréthercétone (PEEK).
Alternativement, ce fourreau pourrait être rigide en étant réalisé par exemple en Titane ou en Polyétheréthercétone (PEEK). Il comporterait alors un filetage simple ou un système de serrage à baïonnette pour être fixé sur la jupe de support du connecteur et/ou la bride de renfort de l'insert.

De préférence, ce fourreau amovible comporte une chambre de purge, le clapet anti-retour étant placé à l'extrémité proximale de ce fourreau.
Cette chambre de purge est avantageusement dimensionnée pour accueillir la tête d'un outil de carottage.
Ainsi, alors que l'extrémité proximale de ce fourreau comporte un clapet anti-retour, son extrémité distale est ouverte afin de pouvoir être introduite et entourer au moins l'extrémité proximale de l'insert.

Avantageusement, le fourreau amovible comporte un orifice de purge pour l'évacuation de l'air, un élément de fermeture permettant d'obturer cet orifice pour assurer l'étanchéité nécessaire.

La présente invention concerne également un montage pour le traitement de maladie cardiaque comprenant un dispositif d'ancrage tel que décrit précédemment et une pompe cardiaque comprenant un carter configuré pour être introduit au travers du second orifice défini par l'insert tout en assurant une étanchéité avec un ou des éléments d'étanchéité portés par l'insert.

De préférence, cette pompe cardiaque comporte une turbine mobile en rotation par rapport au carter et un inducteur.

### Brève description des dessins

D'autres avantages, buts et caractéristiques particulières de la présente invention ressortiront de la description qui va suivre, faite, dans un but explicatif et nullement limitatif, en regard des dessins annexés, dans lesquels:
**Fig. 1**

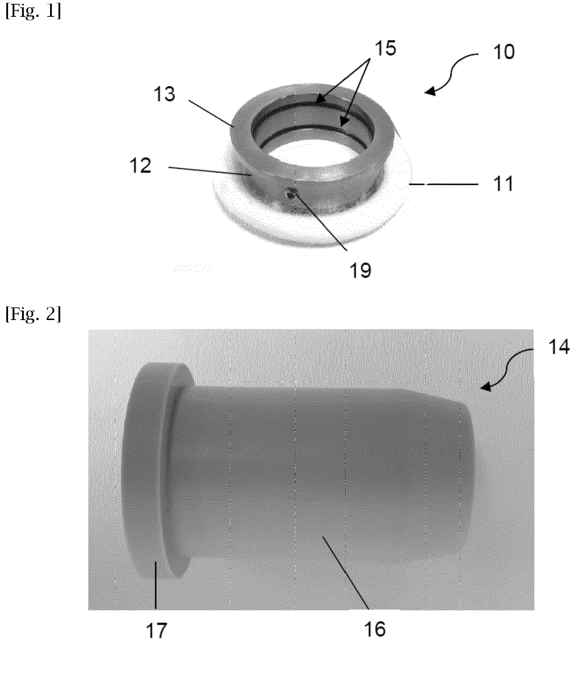

   [Fig. 1] est une vue en perspective d'un connecteur d'un dispositif d'ancrage selon un mode de réalisation particulier de la présente invention ;
**Fig. 2**
   [Fig. 2] est une vue de face de l'insert du dispositif d'ancrage de la Fig. 1 ;
**Fig. 3**

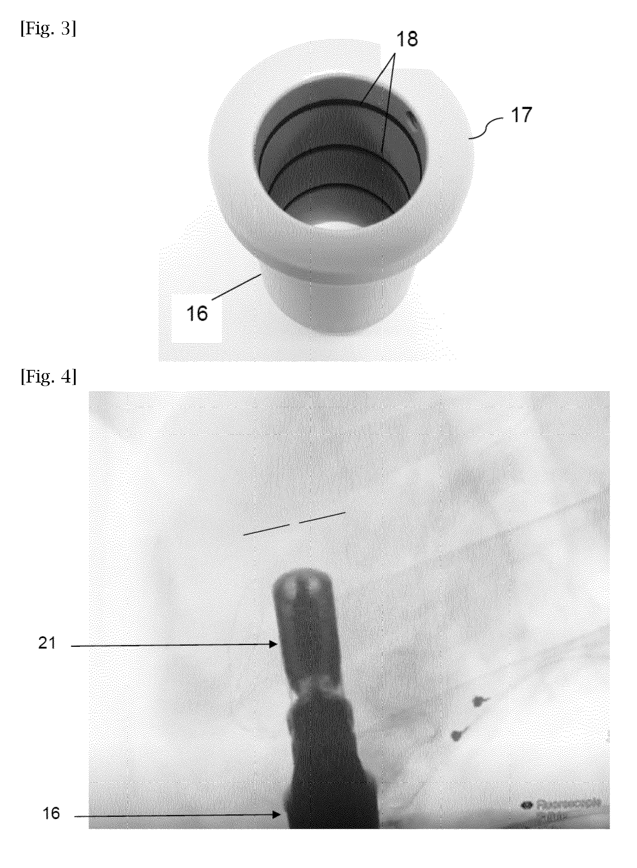

   [Fig. 3] est une vue en perspective et depuis l'extrémité proximale de l'insert du dispositif d'ancrage de la Fig. 1, montrant des éléments d'étanchéité et un orifice traversant dans la bride permettant le passage d'une vis de serrage ;
**Fig. 4**
   [Fig. 4] est une radiographie d'un cœur sur lequel est monté un dispositif d'ancrage de la Fig. 1 et une pompe cardiaque pour assister ce dernier ;
**Fig. 5**

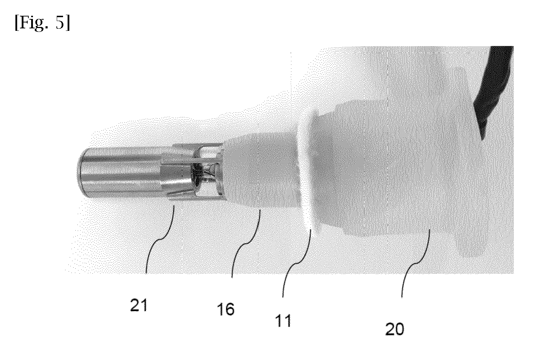

   [Fig. 5] montre un ensemble de pose dans lequel une pompe cardiaque est introduite.

### Description des modes de réalisation

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

Tout d'abord, on note que les figures ne sont pas à l'échelle.

Les Figures 1 à 3 illustrent de manière schématique un dispositif d'ancrage selon un mode de réalisation particulier de la présente invention.

Ce dispositif d'ancrage comporte un connecteur 10 pouvant être monté sur une paroi ventriculaire d'un cœur d'un patient dont on cherche à traiter une insuffisance cardiaque.

Ce connecteur 10 comprend une bride d'assemblage 11, une projection tubulaire 12 s'étendant axialement à partir de cette bride d'assemblage et une bride d'appui 13.

Cette bride d'assemblage 11 est ici destinée à être fixée à la paroi ventriculaire par suture. Elle est avantageusement réalisée dans un matériau facilitant la croissance ou la fixation de cellules afin de renforcer son assemblage avec la paroi ventriculaire.

La projection tubulaire 12 et la bride d'appui 13 sont réalisées d'une seule pièce dans un matériau rigide non thrombogène, tel qu'en titane.

Cette projection tubulaire 12 ainsi que la bride d'appui 13 sont destinées à être placées à l'extérieur du cœur lorsque la bride d'assemblage 11 est assemblée à la paroi ventriculaire, ce connecteur définissant une première ouverture pour le passage d'une partie du corps d'un insert 14.

Des éléments d'étanchéité 15 comprenant des joints toriques sont portés par la projection tubulaire 12 afin d'assurer l'étanchéité de l'assemblage entre le connecteur 10 et l'insert 14.

Cet insert 14 qui est entièrement rigide, comporte une partie tubulaire 16 et une bride de renfort 17. Seule la partie tubulaire 16 de cet insert 14 est destinée à être introduite en partie au travers de la paroi ventriculaire et à être en contact avec cette paroi ventriculaire.
Cet insert 14 est ici réalisé en Polyétheréthercétone (PEEK), mais il pourrait encore être réalisé en Titane.
La surface externe de l'insert 14 peut présenter un relief de surface tel qu'un ou plusieurs maillages, pour favoriser l'adhérence du tissu myocardique et favoriser l'endothélisation, c'est-à-dire le recouvrement tissulaire de l'insert à l'intérieur du ventricule.

L'extrémité libre de cette partie tubulaire 16, ou encore l'extrémité distale de cet insert 14, est chanfreinée pour faciliter son introduction au travers d'une ouverture réalisée dans la paroi ventriculaire au moyen d'un outil de carottage (non représenté).

Cet insert 14 creux définit une seconde ouverture pour le passage du corps d'une pompe cardiaque. Des rainures recevant des joints toriques 18 sont placées dans la surface intérieure de l'insert 14 délimitant la seconde ouverture pour coopérer avec la surface externe du carter d'une pompe cardiaque de manière à assurer l'étanchéité requise. Ces joints toriques 18 pourraient également être placés au niveau de la surface externe de l'insert 14, par exemple sur la partie tubulaire 16 au pied de la bride de renfort 17.

L'extrémité distale de cet insert 14 est destinée à être placée à l'intérieur du cœur tandis que son extrémité proximale, définie par la bride de renfort 17, est destinée à être placée à l'extérieur du cœur lorsqu'il est monté sur le connecteur 10.

La projection tubulaire 12 du connecteur 10 comporte un orifice traversant et une vis de serrage 19 insérée dans cet orifice pour permettre de verrouiller/déverrouiller en position le corps de l'insert 14 par rapport au connecteur 10, lorsque cet insert 14 a été introduit dans le connecteur 10 au travers de la première ouverture.

Ce dispositif d'ancrage particulièrement simple permet par son ancrage dans une ouverture réalisée dans la paroi ventriculaire d'un cœur, de recevoir et supporter le corps d'une pompe cardiaque servant à assister ce cœur défaillant.

Pour assurer la manipulation d'une pompe cardiaque 21, et notamment son introduction dans le dispositif d'ancrage, un fourreau 20 amovible comportant un clapet anti-retour, ou vanne unidirectionnelle, est mis en œuvre pour obturer la seconde ouverture définie par l'insert 14.

Le clapet anti-retour permet l'introduction et le retrait du corps d'une pompe cardiaque ou d'un outil de carottage avec des pertes de sang minimales.

Ce fourreau 20 est réalisé dans un matériau flexible tel qu'en silicone, pour permettre son introduction autour des brides d'appui 13 et de renfort 17. Ce fourreau 20 est configuré pour recouvrir de manière étanche ces éléments.

Il comporte une chambre de purge (non représentée) dont la dimension permet notamment de loger la tête de l'outil de carottage après découpe de la paroi ventriculaire pour former une ouverture.

## Revendications

1. Ensemble de pose d'une pompe cardiaque sur un cœur comprenant un dispositif médical d'ancrage d'une pompe cardiaque dans une ouverture d'une paroi ventriculaire d'un cœur, comprenant un connecteur (10) destiné à être monté sur ladite paroi ventriculaire, dans lequel:
- ledit connecteur (10) étant constitué d'une bride d'assemblage (11) et d'une jupe de support (12) s'étendant à partir de cette bride d'assemblage (11), cette bride d'assemblage étant destinée à être fixée extérieurement à la paroi ventriculaire de sorte que ladite jupe de support (12) est placée à l'extérieur du cœur lorsque la bride d'assemblage (11) est assemblée à la paroi ventriculaire,
- ledit dispositif médical d'ancrage comprend également un insert (14), ce connecteur (10) définissant une première ouverture pour le passage d'une partie du corps de cet insert (14),
- cet insert (14) étant entièrement rigide et définissant une seconde ouverture pour le passage du corps de la pompe cardiaque, cet insert (14) comprenant une extrémité distale destinée à être placée à l'intérieur du cœur et une extrémité proximale destinée à être placée à l'extérieur du cœur lorsque cet insert (14) est monté sur le connecteur (10),
- au moins un élément de verrouillage/déverrouillage (19) en position de l'insert (14) par rapport au connecteur (10), lorsque cet insert (14) a été introduit dans le connecteur (10),
l' ensemble étant **caractérisé en ce qu'**il comprend
- un fourreau (20) amovible comportant un clapet anti-retour, ou vanne unidirectionnelle, ce fourreau (20) étant destiné à être monté sur au moins l'extrémité proximale de l'insert (14) pour prévenir toute perte de sang lors de l'insertion ou du retrait d'un outil ou d'une pompe cardiaque.

2. Ensemble de pose d'une pompe cardiaque sur un cœur selon la revendication 1, **caractérisé en ce que** ce connecteur (10) comprenant une bride de renfort placée à son extrémité proximale, l'insert (14) comporte également une bride de renfort, lesdites brides de renfort étant destinées à être plaquées l'une contre l'autre lorsque l'insert (14) est monté sur le connecteur (10).

3. Ensemble de pose d'une pompe cardiaque sur un cœur selon la revendication 2, **caractérisé en ce que** l'élément de verrouillage/déverrouillage est un élément de serrage traversant latéralement la bride de renfort du connecteur (10) pour serrer le corps de l'insert (14) ou un élément de fixation destiné à assembler ensemble les brides de renfort du connecteur (10) et de l'insert (14).

4. Ensemble de pose d'une pompe cardiaque sur un cœur selon l'une quelconque des revendications 1 à 3, **caractérisé en ce que** l'insert (14) rigide présente une portion tubulaire dont la dimension longitudinale permet à l'extrémité distale de cet insert (14) d'être placée à l'intérieur de la cavité cardiaque lorsque l'insert (14) est monté sur le connecteur (10).

5. Ensemble de pose d'une pompe cardiaque sur un cœur selon l'une quelconque des revendications précédentes, **caractérisé en ce que** le connecteur (10) et/ou l'insert (14) comportent une ou plusieurs rainures recevant chacune un joint d'étanchéité et **en ce que** l'insert (14) comprend un ou plusieurs éléments d'étanchéité pour assurer un assemblage étanche entre cet insert (14) et le corps de pompe destiné à être inséré dans la seconde ouverture.

6. Ensemble de pose d'une pompe cardiaque sur un cœur selon l'une quelconque des revendications précédentes, **caractérisé en ce que** la bride d'assemblage (11) est réalisée dans un matériau permettant de promouvoir la croissance ou la fixation de cellules afin de renforcer son assemblage avec la paroi ventriculaire.

7. Ensemble de pose d'une pompe cardiaque sur un cœur selon l'une quelconque des revendications précédentes, **caractérisé en ce que** l'extrémité distale de l'insert (14) est chanfreinée pour faciliter son insertion au travers d'un orifice réalisé dans la paroi ventriculaire.

8. Ensemble de pose d'une pompe cardiaque sur un cœur selon l'une quelconque des revendications précédentes, **caractérisé en ce que** l'insert (14) comporte un élément de fixation pour bloquer en position le corps de pompe cardiaque lorsque cette dernière a été introduite dans la seconde ouverture.

9. Ensemble de pose selon l'une quelconque des revendications précédentes, **caractérisé en ce que** ce fourreau (20) amovible est réalisé dans un matériau flexible afin qu'il puisse recouvrir de manière étanche au moins l'extrémité proximale de l'insert (14).

10. Ensemble de pose selon l'une quelconque des revendications précédentes, **caractérisé en ce que** le fourreau (20) amovible comporte une chambre de purge, le clapet anti-retour étant placé à l'extrémité proximale de ce fourreau (20).

11. Ensemble de pose selon l'une quelconque des revendications précédentes, **caractérisé en ce que** le fourreau (20) amovible comporte un orifice de purge pour l'évacuation de l'air, un élément de fermeture permettant d'obturer cet orifice.

## Patentansprüche

1. Anordnung zum Anordnen einer Herzpumpe an einem Herzen, umfassend eine medizinische Verankerungsvorrichtung einer Herzpumpe in einer Öffnung einer Ventrikelwand eines Herzens, umfassend einen Konnektor (10), welcher dazu bestimmt ist, an der Ventrikelwand montiert zu werden,
wobei:
- der Konnektor (10) aus einem Verbindungsflansch (11) und einer Stützmanschette (12) besteht, welche sich von diesem Verbindungsflansch (11) erstreckt, wobei dieser Verbindungsflansch dazu bestimmt ist, außen an der Ventrikelwand derart befestigt zu werden, dass die Stützmanschette (12) außerhalb des Herzens platziert wird, wenn der Verbindungsflansch (11) mit der Ventrikelwand verbunden wird,
- die medizinische Verankerungsvorrichtung ebenfalls einen Einsatz (14) umfasst, wobei dieser Konnektor (10) eine erste Öffnung für den Durchgang eines Teils des Körpers dieses Einsatzes (14) definiert,
- dieser Einsatz (14) vollständig starr ist und eine zweite Öffnung für den Durchgang des Körpers der Herzpumpe definiert, wobei dieser Einsatz (14) ein distales Ende, welches dazu bestimmt ist, in dem Inneren des Herzens platziert zu werden, und ein proximales Ende umfasst, welches dazu bestimmt ist, außerhalb des Herzens platziert zu werden, wenn dieser Einsatz (14) an dem Konnektor (10) montiert ist,
- mindestens ein Verriegelungs-/Entriegelungselement (19) an der Position des Einsatzes (14) in Bezug auf den Konnektor (10), wenn dieser Einsatz (14) in den Konnektor (10) eingeführt worden ist,
wobei die Anordnung **dadurch gekennzeichnet ist, dass** sie umfasst:
- eine abnehmbare Hülle (20), welche ein Rückschlagventil oder Einwegventil aufweist, wobei diese Hülle (20) dazu bestimmt ist, mindestens an dem proximalen Ende des Einsatzes (14) montiert zu werden, um jeden Blutverlust bei dem Einführen oder Entnehmen eines Werkzeugs oder einer Herzpumpe zu verhindern.

2. Anordnung zum Anordnen einer Herzpumpe an einem Herzen nach Anspruch 1, **dadurch gekennzeichnet, dass** dieser Konnektor (10) einen Verstärkungsflansch umfasst, welcher an seinem proximalen Ende platziert ist, wobei der Einsatz (14) ebenfalls einen Verstärkungsflansch aufweist, wobei die Verstärkungsflansche dazu bestimmt sind, aneinander angedrückt zu werden, wenn der Einsatz (14) an dem Konnektor (10) montiert wird.

3. Anordnung zum Anordnen einer Herzpumpe an einem Herzen nach Anspruch 2, **dadurch gekennzeichnet, dass** das Verriegelungs-/Entriegelungselement ein Klemmelement ist, welches den Verstärkungsflansch des Konnektors (10) seitlich durchquert, um den Körper des Einsatzes (14) zu klemmen, oder ein Befestigungselement, welches dazu bestimmt ist, die Verstärkungsflansche des Konnektors (10) und des Einsatzes (14) miteinander zu verbinden.

4. Anordnung zum Anordnen einer Herzpumpe an einem Herzen nach einem der Ansprüche 1 bis 3, **dadurch gekennzeichnet, dass** der starre Einsatz (14) einen rohrförmigen Abschnitt aufweist, dessen Längsabmessung es dem distalen Ende dieses Einsatzes (14) ermöglicht, in dem Inneren der Herzhöhle platziert zu werden, wenn der Einsatz (14) an dem Konnektor (10) montiert wird.

5. Anordnung zum Anordnen einer Herzpumpe an einem Herzen nach einem der vorangegangenen Ansprüche, **dadurch gekennzeichnet, dass** der Konnektor (10) und/oder der Einsatz (14) eine oder mehrere Nuten aufweisen, welche jeweils eine Dichtung aufnehmen, und dass der Einsatz (14) ein oder mehrere Dichtungselemente umfasst, um eine dichte Verbindung zwischen diesem Einsatz (14) und dem Pumpenkörper zu gewährleisten, welcher dazu bestimmt ist, in die zweite Öffnung eingeführt zu werden.

6. Anordnung zum Anordnen einer Herzpumpe an einem Herzen nach einem der vorangegangenen Ansprüche, **dadurch gekennzeichnet, dass** der Verbindungsflansch (11) aus einem Material hergestellt ist, welches das Wachstum oder die Befestigung von Zellen fördert, um seine Verbindung mit der Ventrikelwand zu verstärken.

7. Anordnung zum Anordnen einer Herzpumpe an einem Herzen nach einem der vorangegangenen Ansprüche, **dadurch gekennzeichnet, dass** das distale Ende des Einsatzes (14) abgeschrägt ist, um sein Einführen durch eine Öffnung zu erleichtern, welche in der Ventrikelwand ausgebildet ist.

8. Anordnung zum Anordnen einer Herzpumpe an einem Herzen nach einem der vorangegangenen Ansprüche, **dadurch gekennzeichnet, dass** der Einsatz (14) ein Befestigungselement aufweist, um den Körper der Herzpumpe in Position zu blockieren, wenn diese Letztere in die zweite Öffnung eingeführt worden ist.

9. Anordnung zum Anordnen nach einem der vorangegangenen Ansprüche, **dadurch gekennzeichnet, dass** diese abnehmbare Hülle (20) aus einem flexiblen Material hergestellt ist, damit sie mindestens das proximale Ende des Einsatzes (14) dicht abdecken kann.

10. Anordnung zum Anordnen nach einem der vorangegangenen Ansprüche, **dadurch gekennzeichnet, dass** die abnehmbare Hülle (20) eine Entlüftungskammer aufweist, wobei das Rückschlagventil an dem proximalen Ende dieser Hülle (20) platziert ist.

11. Anordnung zum Anordnen nach einem der vorangegangenen Ansprüche, **dadurch gekennzeichnet, dass** die abnehmbare Hülle (20) eine Entlüftungsöffnung für die Evakuierung der Luft aufweist, wobei ein Verschlusselement ermöglicht, diese Öffnung zu verschließen.

## Claims

1. An assembly for fitting a cardiac pump in place on a heart, comprising a medical device for anchoring a cardiac pump in an opening of a ventricular wall of a heart, comprising a connector (10) intended to be mounted on said ventricular wall, wherein:
- said connector (10) including a joining flange (11) and a support skirt (12) extending from this joining flange (11), said joining flange being intended to be fixed outside the ventricular wall so that said support skirt (12) is placed outside the heart when the joining flange (11) is joined to the ventricular wall,
- said medical anchoring device also comprises an insert (14), this connector (10) defining a first opening for the passage of a part of the body of this insert (14),
- this insert (14) being entirely rigid and defining a second opening for the passage of the body of the cardiac pump, this insert (14) comprising a distal end intended to be placed inside the heart and a proximal end intended to be placed outside the heart when this insert (14) is mounted on the connector (10),
- at least one element (19) for positionally locking/ unlocking the insert (14) with respect to the connector (10), when this insert (14) has been introduced into the connector (10), the assembly being **characterized in that** it comprises a removable sheath (20) having a non-return valve, or one-way valve, this sheath (20) being intended to be mounted on at least the proximal end of the insert (14) in order to prevent any loss of blood during the insertion or removal of a tool or of a cardiac pump.

2. The assembly for fitting a cardiac pump in place on a heart as claimed in claim 1, **characterized in that**, with this connector (10) comprising a reinforcing flange placed at its proximal end, the insert (14) also has a reinforcing flange, said reinforcing flanges being intended to be pressed against each other when the insert (14) is mounted on the connector (10).

3. The assembly for fitting a cardiac pump in place on a heart as claimed in claim 2, **characterized in that** the locking/unlocking element is a clamping element passing laterally through the reinforcing flange of the connector (10) in order to clamp the body of the insert (14), or a fixing element intended to join together the reinforcing flanges of the connector (10) and of the insert (14).

4. The assembly for fitting a cardiac pump in place on a heart as claimed in any one of claims 1 through 3, **characterized in that** the rigid insert (14) has a tubular portion, the longitudinal dimension of which allows the distal end of this insert (14) to be placed inside the heart cavity when the insert (14) is mounted on the connector (10).

5. The assembly for fitting a cardiac pump in place on a heart as claimed in any one of the preceding claims, **characterized in that** the connector (10) and/or the insert (14) comprise one or more grooves, each of these receiving a seal, and **in that** the insert (14) comprises one or more sealing elements to ensure a sealed join between this insert (14) and the pump body intended to be inserted into the second opening.

6. The assembly for fitting a cardiac pump in place on a heart as claimed in any one of the preceding claims, **characterized in that** the joining flange (11) is made of a material making it possible to promote the growth or the attachment of cells, in order to reinforce its join with the ventricular wall.

7. The assembly for fitting a cardiac pump in place on a heart as claimed in any one of the preceding claims, **characterized in that** the distal end of the insert (14) is chamfered in order to facilitate its insertion through an orifice formed in the ventricular wall.

8. The assembly for fitting a cardiac pump in place on a heart as claimed in any one of the preceding claims, **characterized in that** the insert (14) has a fixing element for locking the cardiac pump body in position when the latter has been introduced into the second opening.

9. The assembly for fitting a cardiac pump in place on a heart as claimed in any one of the preceding claims, **characterized in that** this removable sheath (20) is made of a flexible material so that it can sealingly cover at least the proximal end of the insert (14).

10. The assembly for fitting a cardiac pump in place on a heart as claimed in any one of the preceding claims, **characterized in that** the removable sheath (20) has a purge chamber, the non-return valve being placed at the proximal end of this sheath (20).

11. The assembly for fitting a cardiac pump in place on a heart as claimed in any one of the preceding claims, **characterized in that** the removable sheath (20) comprises a purge orifice for the evacuation of the air, a closure element making it possible to close this orifice.
